# EUROPÄISCHE PATENTANMELDUNG

(11) **EP 4 176 833 A1**
(43) Veröffentlichungstag der Anmeldung: **10.05.2023**
(21) Anmeldenummer: 22205321.7
(22) Anmeldetag: 03.11.2022
(51) Int. Cl.: A61B 34/30

(54) **ABWINKELBARER ROBOTERARM FÜR DIE MINIMAL-INVASIVE CHIRURGIE, CHIRURGIEROBOTER UND VERFAHREN ZUM HERSTELLEN**

(30) Priorität: 05.11.2021 DE 102021128809
(71) Anmelder: Technische Universität Berlin, 10623 Berlin (DE)
(72) Erfinder: FRITSCH, Sven, 13435 Berlin (DE); OBERSCHMIDT, Dirk, 16515 Oranienburg (DE)
(74) Vertreter: Bittner, Thomas L.

(57) **Zusammenfassung**

Gemäß der Offenbarung ist ein abwinkelbarer Roboterarm für die minimal-invasive Chirurgie geschaffen. Der Roboterarm weist ein proximales Armsegment (1), welches sich entlang einer Längsachse des Roboterarms erstreckt, und ein distales Armsegment (2) auf, welches mit dem proximalen Armsegment (1) um eine zu der Längsachse orthogonale Schwenkachse schwenkbar verbunden ist. Das distale Armsegment (2) weist ein Ritzelsegment auf, welches mit in Form eines Ritzels um die Schwenkachse angeordneten Ritzelzähnen gebildet ist. Ein Zahnstangenelement (12) ist an dem proximalen Armsegment (1) derart angeordnet, dass das Zahnstangenelement (12) in Längsrichtung des Zahnstangenelements (12) parallel zu der Längsachse mittels einer Translation beweglich ist und an dem Zahnstangenelement (12) gebildete Stangenzähne mit den Ritzelzähnen in Eingriff stehen, so dass eine Bewegung des Zahnstangenelements (12) in der Längsrichtung über die Stangenzähne und die Ritzelzähne in eine Rotationsbewegung des distalen Armsegments (2) um die Schwenkachse gewandelt wird und das distale Armsegment (2) relativ zu dem proximalen Armsegment (1) um die Schwenkachse verschwenkt. Weiterhin sind ein Verfahren zum Herstellen eines abwinkelbaren Roboterarms sowie ein Chirurgieroboter bereitgestellt.

## Beschreibung

Die Erfindung betrifft einen abwinkelbaren Roboterarm für die minimal-invasive Chirurgie, einen Chirurgieroboter sowie ein Verfahren zum Herstellen eines abwinkelbaren Roboterarms.

### Hintergrund

Im Bereich der Chirurgie kommen zunehmend Roboter zum Einsatz. Hierbei kann es sich um sogenannte Telemanipulatoren handeln, bei welchen robotische Systeme eine durch eine die Operation durchführende Person vorgegebene Bewegung direkt ausführen. Alternativ können Roboter im engeren Sinne eingesetzt werden, welche Bewegung zumindest teilautonom aufgrund vorheriger Vorgaben durch einen Nutzer und / oder von Sensordaten ausführen.

Insbesondere werden Chirurgieroboter in der minimal-invasiven Chirurgie (MIC bzw. minimally invasive surgery, MIS) eingesetzt, bei welcher der Zugang zum Operationssitus über kleine Einschnitte und / oder natürliche Körperöffnungen erfolgt. Hierbei können Chirurgieroboter dazu dienen, eine durch beengte Zugänge eingeschränkte Beweglichkeit einer operierenden Person zumindest teilweise zu kompensieren. Dies ist insbesondere bei sogenannten Single-Port-Eingriffen von Bedeutung, bei denen der Zugang zum Operationssitus über einen einzigen Zugang erfolgt. So wird bei der Single-Port-Laparoskopie (SPL) ein einziger Schnitt durch den Bauchnabel gesetzt, um durch diesen alle für die Operation benötigten Instrumente in die Bauchhöhle einzuführen.

Bei Roboterarmen für Chirurgieroboter kann unterschieden werden zwischen diskreten und flexiblen Roboterstrukturen. Während diskrete Roboter auf hintereinandergeschalteten Gelenken beruhen, die durch starre Verbindungen miteinander verbunden sind, sind mit flexiblen Robotern kontinuierliche Krümmungen möglich, beispielsweise durch einen gekrümmten Draht.

Bei seriellen Abfolgen von Gelenken und starren Verbindungen im Sinne einer diskreten Roboterstruktur ist es beispielsweise bekannt, eine Winkelverstellung durch Drähte zu erreichen, welche zur Übertragung von Zug- und ggf. Schubkräften genutzt werden, um die Gelenke rotieren lassen, vgl. beispielsweise Thant, Z.M. et al.: Ergonomie Master Controller for Flexible Endoscopic Gastrointestinal Robot Manipulator, Intl. Conf. on Biomedical and Pharmaceutical Engineering, 2006. Alternativ kann eine Winkeleinstellung über eingebettete Elektromotoren erfolgen, die über eine Zahnrad-Band-Kombination eine Neigung bewerkstelligen (vgl. Vitiello, Valentina et al.: Emerging Robotic Platforms for Minimally Invasive Surgery, IEEE Reviews in Biomedical Engineering, 2013, Bd. 6). oder mittels eines Mechanismus aus ineinanderlaufenden Wellen (s. Piccigallo, Marco et al.: Design of a Novel Bimanual Robotic System for Single-Port Laparoscopy, IEEE/ASME Transactions on Mechatronics, 2010, Bd. 15, 6). Ein Winkeleinstell-Mechanismus nach Lehman, Amy C. et al.: Natural orifice cholecystectomy using a miniature robot, Surgical Endoscopy, 2009, setzt sich zusammen aus zwei sogenannten Linkages, die über ein in einem Mittelelement eingebetteten Elektromotor zwei Seitenelemente rotieren lassen.

Bei flexiblen Robotern hat sich insbesondere die Aktuation mit NiTi-Drähten durchgesetzt. Beispielsweise können über solche Drähte Klappen an Roboterarmen geöffnet werden, welche Instrumente bzw. Instrumentenarme ablenken (vgl. Bardou, Berengere et al.: Design of a telemanipulated system for transluminal surgery, 31st Annual International Conference of the IEEE EMBS, 2009; Swanstrom, Lee et al.: Development of a New Access Device for Transgastric Surgery, The Society for Surgery of the Alimentary Tract, 2005; sowie De Donno, Antonio etal.: Introducing STRAS: a New Flexible Robotic System for Minimally Invasive Surgery, 2013 IEEE International Conference on Robotics and Automation (ICRA), 2013). Eine Winkelverstellung kann auch über ein gewickeltes Kabel erreicht werden, welches im Gegensatz zu einem NiTi-Draht nicht auf Zug und Druck belastbar ist, sondern nur auf Zug. Ein solches Kabel kann mit einer Umdrehung um einen Pfosten gewickelt sein. Auf einer gegenüberliegenden Seite kann das Kabel in die entgegengesetzte Richtung um einen zweiten Pfosten gewickelt sein, so dass, wenn das Kabel auf einer Seite gezogen wird, während das Kabel auf der anderen Seite nachlaufen gelassen wird, sich ein Roboterarm in die entsprechende Richtung abwinkelt. So ist eine Rotation in beide Richtungen möglich (vgl. zum Beispiel Can, Salman et al.: Design, Development and Evaluation of a Highly Versatile Robot Platform for Minimally Invasive Single-Port Surgery, The Fourth IEEE RAS/EMBS International Conference on Biomedical Robotics and Biomechatronics, 2012.).

Bei einem Konzept mit einer Vielzahl an Segmenten (beispielsweise gemäß Ouyang, Bo et al.: Design of a three-segment continuum robot for minimally invasive surgery, Robotics and Biomemetics, 2016) kann eine Winkelverstellung erreicht werden, indem das erste Segment, also das Segment, welches einer Grundplatte am nächsten ist, gebogen wird, während das zweite Segment in die entgegengesetzte Richtung gebogen wird. Es bestehen ähnliche Konzepte, die meistens lediglich zwei serielle Segmente aufweisen (vgl. Xu, Kai et al.: Development of the SJTU Unfoldable Robotic System (SURS) for Single Port Laparoscopy, IEEE/ASME Transactions on Mechatronics 1, 2015). Insbesondere kann es ermöglicht sein, eine S-Kurve zu bilden. Eine Winkelverstellung kann insbesondere durch die gezielte Krümmung einzelner Segmente erfolgen, die aus NiTi-Draht bestehen. Zusätzlich kann vorgesehen sein, mehrere Roboterarme eines Roboters auseinanderzudrücken, beispielsweise mittels eines Parallelogramm-Mechanismus. In einem beispielhaften Parallelogramm-Mechanismus (s. Xu, Kai et al.: System Design of an Insertable Robotic Effector Platform for Single Port Access (SPA) Surgery, The 2009 IEEE/RSJ International Conference on Intelligent Robots and Systems, 2009) wird durch Zurückziehen einer Schiene eine Aufrichtung des ParallelogrammArms erzielt, während eine Verschiebung der Schiene nach vorne zu einem Anlegen des flexiblen Roboter-Teils führt.

Eine weitere Möglichkeit, Winkel bei flexiblen Robotern einzustellen, ist die Verwendung von Kammern, die mit einem Fluid befüllt werden können (z.B. kompressierte Luft oder Wasser). Ein solches Konzept ist beispielsweise bekannt aus Chen, G. et al.: Development and kinematic analysis of a siliconerubber bending tip for colonoscopy, Proceedings of the 2006 IEEE/RSJ International Conference on Intelligent Robots and Systems, 2006.

In robotischen Systemen ist verbreitet eine Wandlung von Linear- in Rotationsbewegungen oder umgekehrt erforderlich. Hierbei ist es bekannt, für die Erzeugung einer Linear Bewegung aus einer Rotationsbewegung Zahnstangen-Ritzel-Paarungen einzusetzen, in denen das Ritzel, welches auf einer Motorwelle montiert ist, die Zahnstange antreibt, um eine Linearbewegung zu erzielen. Dabei können eine hohe Laufruhe, Positioniergenauigkeit und Vorschubkraft erzielt werden. Bei Maschinen (beispielweise Baumaschinen, Werkzeugmaschinen, Biegemaschinen, Formträgern, Gießerei-, Bergbau-, Land- und Verpackungsmaschinen), die eine Linearbewegung mit großer Kraft bereitstellen, insbesondere mittels Öldruck, sind Kolbendrehantriebe bekannt, bei denen Öldruck das Verschieben von Zahnstangen bewirkt, wobei die Linearbewegung der Zahnstangen über eine Verzahnung in eine Drehbewegung des Ritzels umgewandelt wird.

Aus dem Dokument WO 03 / 013349 A1 ist es bekannt, eine Spitze einer Bildsonde mittels einer Zahnstangen-Ritzel-Paarung abzuwinkeln. Das Dokument DE 10 2015 114 742 B4 beschreibt eine Bewegung von Zangenbacken eines endoskopischen Instruments über eine Zahnstange.

Das Dokument DE 10 2010 034 380 A1 offenbart einen Gelenkabschnitt eines Endoskopschafts zur abwinkelbaren Verbindung eines distalen Teils des Endoskopschafts mit einem proximalen Teil des Endoskopschafts. Der distale Schaftteil ist über zwei unterschiedlich lange starre Gelenkstäbe mit dem proximalen Schaftteil verbunden, die jeweils mit einem Gelenk am distalen und am proximalen Schaftteil ansetzen. Der Artikel "Besserer Durchblick für den Chirurgen" (abrufbar https://robotik-produktion.de/allgemein/besserer-durchblick-fuerden-chirurgen/) beschreibt einen Roboterarm für das Halten und Bewegen eines Endoskops während einer Operation.

### Zusammenfassung

Aufgabe der Erfindung ist es, verbesserte Technologien für Chirurgieroboter mit abwinkelbaren Roboterarmen anzugeben, bei denen insbesondere eine mechanische Komplexität gering ist und ein effizienter Betrieb gewährleistet ist.

Die Aufgabe wird gelöst durch einen abwinkelbaren Roboterarm nach dem unabhängigen Anspruch 1 sowie einen Chirurgieroboter und ein Verfahren zum Herstellen eines abwinkelbaren Roboterarms nach weiteren unabhängigen Ansprüchen. Ausgestaltungen sind Gegenstand von abhängigen Ansprüchen.

Gemäß einem Aspekt ist ein abwinkelbarer Roboterarm für die minimal-invasive Chirurgie geschaffen. Der abwinkelbare Roboterarm weist ein proximales Armsegment, welches sich entlang einer Längsachse des Roboterarms erstreckt, und ein distales Armsegment auf, welches mit dem proximalen Armsegment um eine zu der Längsachse orthogonale Schwenkachse schwenkbar verbunden ist. Das distale Armsegment weist ein Ritzelsegment auf, welches mit in Form eines Ritzels um die Schwenkachse angeordneten Ritzelzähnen gebildet ist. Ein Zahnstangenelement ist an dem proximalen Armsegment derart angeordnet, dass das Zahnstangenelement in Längsrichtung des Zahnstangenelements parallel zu der Längsachse mittels einer Translation beweglich ist, und an dem Zahnstangenelement gebildete Stangenzähne mit den Ritzelzähnen in Eingriff stehen, so dass eine Bewegung des Zahnstangenelements in der Längsrichtung über die Stangenzähne und die Ritzelzähne in eine Rotationsbewegung des distalen Armsegments um die Schwenkachse gewandelt wird und das distale Armsegment relativ zu dem proximalen Armsegment um die Schwenkachse verschwenkt.

Gemäß einem weiteren Aspekt ist ein Chirurgieroboter bereitgestellt, insbesondere für die minimal-invasive Chirurgie, der einen abwinkelbaren Roboterarm gemäß der Offenbarung aufweist.

Nach noch einem Aspekt ist ein Verfahren zum Herstellen eines abwinkelbaren Roboterarms geschaffen. Das Verfahren umfasst die Schritte des Bereitstellens eines proximalen Armsegments, dessen Erstreckungsrichtung eine Längsachse des Roboterarms definiert, des Bereitstellens eines distalen Armsegments mit einem Ritzelsegment, welches mit in Form eines Ritzels um eine Schwenkachse angeordneten Ritzelzähnen gebildet ist, des Verbindens des distalen Armsegments mit dem proximalen Armsegment, derart, dass das distale Armsegment um die Schwenkachse relativ zu dem proximalen Armsegment schwenkbar ist und die Schwenkachse zu der Längsachse orthogonal verläuft, und des Anordnen eines Zahnstangenelements an dem proximalen Armsegment. Das Zahnstangenelement wird an dem proximalen Armsegment derart angeordnet, dass das Zahnstangenelement in Längsrichtung des Zahnstangenelements parallel zu der Längsachse mittels einer Translation beweglich ist, und an dem Zahnstangenelement gebildete Stangenzähne mit den Ritzelzähnen in Eingriff stehen, so dass eine Bewegung des Zahnstangenelements in der Längsrichtung über die Stangenzähne und die Ritzelzähne in eine Rotationsbewegung des distalen Armsegments um die Schwenkachse gewandelt wird und das distale Armsegment relativ zu dem proximalen Armsegment um die Schwenkachse verschwenkt.

Im Sinne der vorliegenden Offenbarung beziehen sich die Begriffe Roboter und Roboterarm sowohl auf Roboter im engeren Sinne, welche autonome oder teil-autonome Bewegungen ausführen, als auch auf Telemanipulationssysteme, bei denen robotische Arme eine direkt von einer bedienenden Person vorgegebene Bewegung ausführen. Begrifflich umfasst sind hierbei auch Kombinationen der beiden Konzepte.

Der Begriff proximal bezeichnet im Zusammenhang mit dem abwinkelbaren Roboterarm und dem Chirurgieroboter eine einem zentralen Hauptteil des Roboters zugewandte Richtung bzw. Anordnung. Der Begriff distal bezeichnet eine dem zentralen Hauptteil abgewandte und dem Operationssitus zugewandte Richtung bzw. Anordnung.

Überraschenderweise wurde festgestellt, dass mit einer Zahnstangen-Ritzel-Paarung gemäß den unabhängigen Ansprüchen, welche durch das Zahnstangenelement und das Ritzelsegment gebildet ist, eine ausreichende Stabilität und Kraftwirkung für die Verwendung in einem abwinkelbaren Roboterarm für die minimal-invasive Chirurgie bereitgestellt werden kann, während gleichzeitig eine für diese Anwendung geeignete Dimensionierung der Komponenten vorgesehen ist. Demgegenüber entspricht es dem Stand der Technik, beispielsweise gemäß den oben genannten Dokumenten WO 03 / 013349 A1 und DE 10 2015 114 742 B4, für eine Verwendung in Roboterarmen der minimal-invasiven Chirurgie ausreichend miniaturisierte Zahnstangen-Ritzel-Paarungen ausschließlich für Anwendungen mit einem geringen Kraftbedarf einzusetzen. Im Vergleich erfordert eine Abwinklung eines Roboterarms, welche auch dann zuverlässig funktionieren muss, wenn der Roboterarm den im Einsatz erwartbaren Lasten ausgesetzt ist, einen hohen Kraftaufwand. Offenbarungsgemäß kann eine Abwinklung eines abwinkelbaren Roboterarms frei von einer Verwendung von Kabeln oder Drähten für diese Abwinklung bereitgestellt sein. Hierbei können Kabel oder Drähte für zusätzliche Bewegungen und / oder Freiheitsgrade des Roboterarms vorgesehen sein.

Die Ritzelzähne können als Ausformungen des distalen Armsegments gebildet sein. In diesem Fall bildet das Ritzelsegment einen Abschnitt des distalen Armsegments. Beispielsweise können die Ritzelzähne mittels eines additiven Verfahrens, insbesondere 3D-Druck, zusammen mit dem distalen Armsegement hergestellt werden, die Ritzelzähne können an das distale Armsegment angeformt sein oder die Ritzelzähne können aus dem distalen Armsegment herausgeformt sein, zum Beispiel mittels spanender Bearbeitung wie Fräsen oder durch Stanzen oder Schneiden, zum Beispiel Laser- oder Wasserstrahlschneiden. Durch eine Bildung der Ritzelzähne als Ausformungen des distalen Armsegments können die Anzahl benötigter Verbindungselemente sowie hiermit verbunden Materialkosten und Montagezeit reduziert sein.

Alternativ können die Ritzelzähne separat hergestellt und an dem distalen Armsegment befestigt sein, beispielsweise mittels Kleben, Schweißen, Löten, Schrauben oder Nieten. Hierbei bildet das Ritzelsegment ein separates Element, das an dem distalen Armsegment angeordnet und an diesem befestigt ist. Insbesondere kann ein Ritzel oder ein Abschnitt eines Ritzels (insbesondere in Form eines Kreissegments mit Zähnen entlang des Umfangs) an dem distalen Armsegment angeordnet und fest mit diesem verbunden sein. Hierdurch kann die Verwendung von Standardbauteilen ermöglicht sein, wodurch die Herstellungskosten reduziert sein können.

Die Ritzelzähne können sich um die Schwenkachse über einen begrenzten Winkelbereich erstrecken. Insbesondere kann hierdurch an dem distalen Armsegment mit dem Ritzelsegment ein Abschnitt eines Ritzels gebildet und / oder angeordnet sein. Der begrenzte Winkelbereich kann einem Bewegungsradius der Abwinklung des Roboterarms entsprechen. Beispielsweise können die Ritzelzähne einen Winkel zwischen einer vollständigen Streckung des distalen Armsegments gegenüber dem proximalen Armsegment (entsprechend 0°) und einem rechten Winkel zwischen proximalem Armsegment und distalem Armsegment (90°) umspannen.

Das distale Armsegment kann ein zweites Ritzelsegment mit um die Schwenkachse angeordneten Ritzelzähnen aufweisen, welches von dem Ritzelsegment entlang der Schwenkachse beabstandet an dem distalen Armsegment angeordnet ist. Insbesondere können die Ritzelsegmente an sich entlang einer durch die Schwenkachse vorgegebenen Richtung gegenüberliegenden Seiten des distalen Armsegments angeordnet sein. Hierbei stehen Stangenzähne mit beiden Ritzelsegmenten in Eingriff, um das distale Armsegment zu verschwenken. Durch das Bereitstellen eines zweiten Ritzelsegments, insbesondere an gegenüberliegenden Seiten des distalen Armsegments, kann ein Verklemmen und / oder Verkanten des Abwinkelmechanismus verhindert oder reduziert sein. Weiterhin kann mit einer Ausführung mit einem zweiten Ritzelsegment ein Sicherheitsmechanismus bereitgestellt sein, der nach einem eventuellen Zahnbruch eines der Ritzelsegmente das sichere Anlegen und Entfernen des Roboters durch das verbleibende, noch intakte Ritzelsegment ermöglicht.

Das Zahnstangenelement kann zwei Sätze von Stangenzähnen aufweisen, wobei die zwei Sätze von Stangenzähnen parallel und in Richtung der Schwenkachse beabstandet zueinander angeordnet sind, derart, dass der erste Satz von Stangenzähnen mit den Ritzelzähnen des Ritzelsegments in Eingriff steht und der zweite Satz von Stangenzähnen mit den Ritzelzähnen des zweiten Ritzelsegments in Eingriff steht.

Alternativ kann für Ausführungen mit einem zweiten Ritzelsegment vorgesehen sein, dass das Zahnstangenelement lediglich einen Satz von Stangenzähnen aufweist, wobei die Stangenzähne so breit ausgeführt sind, dass sie mit beiden Ritzelsegmenten in Eingriff stehen, um das distale Armsegment zu verschwenken.

Das Zahnstangenelement kann mit einem proximalen Zahnstangensegment und einem in der Längsrichtung an das proximale Zahnstangensegment anschließendem distalen Zahnstangensegment gebildet sein, wobei das distale Zahnstangesegment in Richtung der Schwenkachse breiter ist als das proximale Zahnstangensegment. Hierbei können die Stangenzähne im Bereich des distalen Zahnstangenelements angeordnet sein. Das proximale Zahnstangesegment kann frei von Stangenzähnen sein. Bei Ausführungen mit zwei Sätzen von Stangenzähnen können die Sätze von Stangenzähnen parallel und in Richtung der Schwenkachse beabstandet zueinander an dem distalen Zahnstangensegment angeordnet sein.

Das proximale Armsegment kann einen entlang der Längsachse verlaufenden Führungskanal aufweisen, in welchem das Zahnstangenelement, insbesondere mit einem proximalen Zahnstangensegment, zumindest abschnittsweise angeordnet und geführt ist. Beispielsweise kann der Führungskanal im Querschnitt eine V-Form oder U-Form aufweisen. Der Führungskanal kann sich über die gesamte Länge des proximalen Armsegments erstrecken. Alternativ kann sich der Führungskanal nur teilweise entlang des proximalen Armsegments erstrecken, wobei die Länge des Führungskanals mindestens einer Länge entspricht, um welche das Zahnstangenelement für eine maximale Abwinklung des distalen Armsegments parallel zu der Längsachse bewegbar ist. In diesem Fall kann das Zahnstangenelement ein vorstehendes Führungselement aufweisen welches in dem Führungskanal angeordnet und in diesem linear geführt ist.

Zusätzlich oder alternativ zu einem Führungskanal des proximalen Armsegments kann das proximale Armsegment ein Armsegment-Führungselement aufweisen, welches vorzugsweise an einem distalen Ende des proximalen Armsegments in der Längsrichtung vorspringend angeordnet ist, insbesondere einstückig an das proximale Armsegment angeformt oder als separates Element gebildet und an dem proximalen Armsegment befestigt ist. Das Armsegment-Führungselement kann als Führungsstab ausgebildet sein. In einer Ausführung des abwinkelbaren Roboterarms mit Armsegment-Führungselement weist das Zahnstangenelement einen Zahnstangenelement-Führungskanal auf in welchem das Armsegment-Führungselement zumindest abschnittsweise angeordnet und geführt ist. Der Zahnstangenelement-Führungskanal kann auf einer den Stangenzähnen gegenüberliegenden Seite des Zahnstangenelements angeordnet sein, insbesondere an einer den Stangenzähnen gegenüberliegenden Seite des distalen Zahnstangenelements. Das Armsegment-Führungselement und der Zahnstangenelement-Führungskanal weisen vorzugsweise einen nicht-runden Querschnitt auf, insbesondere einen rechteckigen Querschnitt.

Durch eine Führung der Relativbewegung zwischen dem proximalen Armsegment und dem Zahnstangenelement mittels des Führungskanals und / oder des Zahnstangenelement-Führungskanals können eine sichere Führung und ein ruhiger Lauf gewährleistet sein, insbesondere dadurch, dass Seitenwände des Führungskanals und / oder des Zahnstangenelement-Führungskanals das Zahnstangenelement bzw. das Armsegment-Führungselement umgreifen. Durch eine Führung im Bereich der bzw. in geringer Entfernung zu den Stangenzähnen können wirkende Radialkräfte und / oder Tangentialkräfte der Zahnstangen-Ritzel-Paarung des abwinkelbaren Roboterarms in den Elementen der Führung aufgenommen werden, wodurch insbesondere ein auf das Zahnstangenelement wirkendes Biegemoment reduziert sein kann. Somit kann eine mechanische Belastung des Zahnstangenelements reduziert sein.

Das distale Armsegment kann zumindest abschnittsweise als flexibles Armsegment gebildet sein, welches entlang seiner Längserstreckung kontinuierlich oder quasi-kontinuierlich abwinkelbar ist. Hierbei kann das distale Armsegment distal eines Bereichs, in dem das Ritzelsegment angeordnet ist gemäß einer flexiblen Roboterstruktur ausgebildet sein, beispielsweise gemäß den Prinzipien flexibler Roboterstrukturen, welche als solche aus dem Stand der Technik bekannt sind, oder gemäß von dem Stand der Technik abweichenden flexiblen Roboterstrukturen.

In einer bevorzugten Ausführung kann das flexible Armsegment mit mindestens drei Zug-Schub-Mitteln gebildet sein, welche entlang einer Längserstreckung des flexiblen Armsegments parallel und entlang der Längserstreckung verschiebbar zueinander angeordnet sind. Jedem Zug-Schub-Mittel kann ein entsprechender Linearaktor zugeordnet sein, welcher eingerichtet ist das betreffende Zug-Schub-Mittel entlang der Längserstreckung des flexiblen Armsegments zu verschieben. Hierbei können die entsprechenden Linearaktoren Teil des abwinkelbaren Roboterarms sein, oder die betreffenden Zug-Schub-Mittel können eingerichtet sein, mit einem entsprechenden Linearaktor funktionsverbunden zu werden, welcher außerhalb des abwinkelbaren Roboterarms angeordnet ist, beispielsweise als Teil eine Chirurgieroboters, welchem der abwinkelbare Roboterarm zugeordnet ist. Ein Linearaktor kann ein Aktor sein, der direkt eine Linearbewegung bereitstellt oder ein Aktor, der eine andere Bewegungsform bereitstellt, beispielsweise eine Rotation, in Verbindung mit einer Mechanik, welche die von dem Aktor bereitgestellte Bewegung in eine Linearbewegung wandelt.

Bei den Zug-Schub-Mitteln kann es sich um Drähte handeln, insbesondere NiTi-Drähte, also Drähte aus einer Nickel-Titan-Legierung. Die Drähte können eine ausreichend hohe Knicksteifigkeit aufweisen, um als Zug-Schub-Mittel sowohl in einem Zug- als auch in einem Schub-Modus zum Einsatz zu kommen. Bei einer Abwinklung bzw. Biegung des flexiblen Armsegments kann die Länge eines bezogen auf den Querschnitt des flexiblen Armsegments zentral angeordneten Zug-Schub-Mittels konstant bleiben, der entsprechende Linearaktor kann sich also nicht bewegen, während einer oder mehrere der verbleibenden Zug-Schub-Mittel bewegt, also entlang der Längserstreckung des flexiblen Armsegments verschoben, werden.

Gemäß einer bevorzugten Ausführung kann das flexible Armsegment mit mindestens fünf Zug-Schub-Mitteln gebildet sein, wobei die oben ausgeführten Ausgestaltungen bezüglich der Zug-Schub-Mittel entsprechend vorgesehen sein können. Insbesondere kann bei einer Abwinklung bzw. Biegung des flexiblen Armsegments die Länge eines bezogen auf den Querschnitt des flexiblen Armsegments zentral angeordneten Zug-Schub-Mittels konstant bleiben, während einer oder mehrere der verbleibenden Zug-Schub-Mittel bewegt, also entlang der Längserstreckung des flexiblen Armsegments verschoben, werden. Hierbei können zwei der nicht-zentralen Zug-Schub-Mittel antagonistisch aktuiert werden, so dass das eine Zug-Schub-Mittel in eine distale Richtung geschoben wird, während das andere Zug-Schub-Mittel auf einer bezogen auf das zentrale Zug-Schub-Mittel gegenüberliegenden Seite in eine proximale Richtung gezogen wird, so dass eine Biegung des flexiblen Armsegments erreicht wird.

Bei einer Ausführung des distalen Armsegments zumindest abschnittsweise als flexibles Armsegment kann das distale Armsegment mit einem festen Segment gebildet sein, welches mit dem proximalen Armsegment um die Schwenkachse schwenkbar verbunden ist und welches das Ritzelsegment aufweist, wobei sich das flexible Armsegment direkt oder mittelbar an das feste Segment anschließt. Hierbei können insbesondere Zug-Schub-Elemente des flexiblen Armsegments durch das proximale Armsegment und das feste Segment des distalen Armsegments geführt sein. Das feste Segment kann Element-Führungskanäle für die Führung der Zug-Schub-Elemente aufweisen. Mittels der Element-Führungskanäle kann eine unterschiedliche Verteilung der Zug-Schub-Elemente in dem distalen Armsegment gegenüber dem proximalen Armsegment erreicht sein. Beispielsweise kann vorgesehen sein, dass die Zug-Schub-Elemente in dem proximalen Armsegment direkt linear nebeneinander angeordnet geführt sind, während sie entlang des distalen Armsegment einem flexiblen Armsegment entsprechend angeordnet sind, insbesondere kreisförmig um ein zentrales Zug-Schub-Element herum. Hierzu kann vorgesehen sein, dass eine Anordnung der Element-Führungskanäle einer Anordnung der Zug-Schub-Elemente in dem distalen Armsegment entspricht, so dass die Zug-Schub-Elemente vor dem Eintreten in die Element-Führungskanäle gegenüber ihrer Anordnung in dem proximalen Armsegment umgeordnet werden. Alternativ können die Element-Führungskanäle so ausgestaltet sein, dass sie eine Umordnung der Zug-Schub-Elemente direkt führen vorgeben. In diesem Fall entspricht die Anordnung von dem proximalen Armsegment zugewandten Eingängen der Element-Führungskanäle einer Anordnung der Zug-Schub-Elemente in dem proximalen Armsegment und eine Anordnung von dem proximalen Armsegment abgewandten Ausgängen der Element-Führungskanäle entspricht einer Anordnung der Zug-Schub-Elemente in dem distalen Armsegment.

Alternativ oder zusätzlich zu einem flexiblen Armsegment kann das distale Armsegment zumindest abschnittsweise als diskret-robotisches Armsegment mit entlang seiner Längserstreckung hintereinander angeordneten Segmenten gebildet sein, wobei das diskret-robotische Armsegment entlang seiner Längserstreckung segmentweise abwinkelbar ist. Hierbei kann das diskret-robotische Armsegment distal eines Bereichs, in dem das Ritzelsegment angeordnet ist gemäß einer diskreten Roboterstruktur ausgebildet sein, beispielsweise gemäß den Prinzipien diskreter Roboterstrukturen, welche als solche aus dem Stand der Technik bekannt sind, oder gemäß von dem Stand der Technik abweichenden diskreten Roboterstrukturen. In Ausführungen, in denen das distale Armsegment sowohl wenigstens ein flexibles Armsegment als auch wenigstens ein diskret-robotisches Armsegment aufweist, können das flexible Armsegment und das diskret-robotische Armsegment entlang der Längserstreckung des distalen Armsegments in geeigneter Reihenfolge hintereinander angeordnet sein, wobei sowohl das flexible Armsegment als auch das diskret-robotische Armsegment distal eines Bereichs ausgebildet sind, in dem das Ritzelsegment angeordnet ist, insbesondere distal eine festen Segments des distalen Armsegments.

Der abwinkelbare Roboterarm kann einen Linearaktor aufweisen, welcher mit dem Zahnstangenelement verbunden und eingerichtet ist, das Zahnstangenelement parallel zu der Längsachse translatorisch vor- und zurückzubewegen. Alternativ hierzu kann das Zahnstangenelement eingerichtet sein, mit einem entsprechenden Linearaktor funktionsverbunden zu werden, welcher außerhalb des abwinkelbaren Roboterarms angeordnet ist, beispielsweise als Teil eine Chirurgieroboters, welchem der abwinkelbare Roboterarm zugeordnet ist. Ein Linearaktor kann hierbei ein Aktor sein, der direkt eine Linearbewegung bereitstellt oder ein Aktor, der eine andere Bewegungsform bereitstellt, beispielsweise eine Rotation, in Verbindung mit einer Mechanik, welche die von dem Aktor bereitgestellte Bewegung in eine Linearbewegung wandelt.

Das distale Armsegment kann mit dem proximalen Armsegment mittels einer Schraubenverbindung um die Schwenkachse schwenkbar verbunden sein. Alternativ oder zusätzlich kann eine andere Verbindungsart, beispielsweise eine Nietverbindung eine Verbindung über ein separates Gelenkbauteil oder eine direkte und rotatorisch bewegliche formschlüssige Verbindung zwischen dem distalen Armsegment und dem proximalen Armsegment vorgesehen sein. Das proximale Armsegment kann mit einer Verbindungs-Ausformung gebildet sein, welche sich zu dem distalen Armsegment erstreckt und mit diesem schwenkbar verbunden ist. Insbesondere kann vorgesehen sein, dass sich die Verbindungs-Ausformung zwischen zwei Ritzelsegmente des distalen Armsegments erstreckt und zwischen diesen schwenkbar fixiert wird. Hierbei können die Ritzelsegmente als Ausformungen von Flankenelementen des distalen Armsegments gebildet sein, und die Verbindungs-Ausformung kann sich zwischen die Flankenelemente erstrecken und zwischen diesen schwenkbar fixiert sein. Die Verbindungs-Ausformung kann einstückig mit dem proximalen Armsegment gebildet oder separat gebildet und an diesem fest angeordnet sein.

Der Chirurgieroboter kann als im Körper eines Patienten montierbarer Roboter ausgebildet sein. Hierbei kann vorgesehen sein, einzelne Elemente des Chirurgieroboters, beispielsweise umfassend mehrere Roboterarme, separat in den Körper des Patienten einzubringen und im Körper des Patienten miteinander zu dem Chirurgieroboter zu verbinden. Bei einer Ausgestaltung des Chirurgieroboters als im Körper eines Patienten montierbarer Roboter kann dadurch, dass der Chirurgieroboter in mehreren separaten Elementen in den Körper des Patienten eingebracht wird, ermöglicht sein, den Chirurgieroboter durch einen kleineren Schnitt in der Bauchdecke des Patienten in den Körper des Patienten einzubringen, als dies anderenfalls möglich wäre. Beispielsweise kann vorgesehen sein, mehrere Roboterarme, vorzugsweise vier Roboterarme in den Körper des Patienten einzubringen und anschließend ein Gehäuse in dem Zugang zum Körper des Patienten anzuordnen. Anschließend können die Roboterarme in dem Gehäuse angeordnet und fixiert werden, um den montierbaren Roboter zu montieren. Hierbei kann ein dichtendes Fixierelement bereitgestellt sein, welches nach dem Anordnen der Roboterarme in dem Gehäuse von außerhalbe des Körper des Patienten in das Gehäuse eingebracht wird und die Roboterarme dichtend und fixierend umschließt.

Im Zusammenhang mit dem Verfahren zum Herstellen eines abwinkelbaren Roboterarms kann das Breitstellen des distalen Armsegments das Ausstanzen eines Blechs und das Abkanten des gestanzten Blechs zu dem distalen Armsegment umfassen. Alternativ kann das Breitstellen des distalen Armsegments das zumindest abschnittsweise Herstellen des distalen Armsegments mittels eines additiven Fertigungsverfahrens umfassen. Beispielsweise kann vorgesehen sein, das distale Armsegment mittels eines 3D-Druck-Verfahrens herzustellen. Hierbei kann insbesondere das Ritzelsegment 3D-gedruckt sein. Alternativ oder zusätzlich kann jedes oder alle der Elemente des abwinkelbaren Roboterarms mittels eines additiven Fertigungsverfahrens, beispielsweise 3D-Druck, hergestellt werden. Entsprechend kann für den abwinkelbaren Roboterarm vorgesehen sein, dass eines, einige oder alle Elemente mittels eines additiven Fertigungsverfahrens, beispielsweise 3D-Druck, hergestellt sind.

Entsprechend erstreckt sich die Offenbarung auf ein Computerprogrammprodukt umfassend Befehle, die bei der Ausführung des Programms auf einer Vorrichtung zur additiven Fertigung, insbesondere einem 3D-Drucker, eine additive Fertigung des abwinkelbaren Roboterarms bzw. dessen Komponenten veranlassen.

Die oben im Zusammenhang mit dem abwinkelbaren Roboterarm erläuterten Ausführungsbeispiele können für den Chirurgieroboter sowie das Verfahren zum Herstellen eines abwinkelbaren Roboterarms entsprechend vorgesehen sein und umgekehrt.

### Beschreibung von Ausführunqsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines abwinkelbaren Roboterarms für die minimal-invasive Chirurgie;
- Fig. 2A: eine schematische Detailansicht des abwinkelbaren Roboterarms gemäß der Fig. 1;
- Fig. 2B: eine weitere schematische Detailansicht des abwinkelbaren Roboterarms gemäß der Fig. 1;
- Fig. 2C: noch eine weitere schematische Detailansicht des abwinkelbaren Roboterarms gemäß der Fig. 1;
- Fig. 3: eine schematische Darstellung eines Zahnstangenelements eines abwinkelbaren Roboterarms;
- Fig. 4A: eine schematische Darstellung eines proximalen Armsegments eines abwinkelbaren Roboterarms;
- Fig. 4B: eine schematische Detailansicht des proximalen Armsegments aus Fig. 4A;
- Fig. 5A: eine schematische Darstellung eines Elements eines distalen Armsegments eines abwinkelbaren Roboterarms;
- Fig. 5B: eine schematische Darstellung des Elements aus Fig. 5A aus einer anderen Perspektive;
- Fig. 5C: eine schematische Schnittdarstellung des Elements aus Fig. 5A;
- Fig. 6A: eine schematische Darstellung eines Teils eines Chirurgieroboters;
- Fig. 6B: eine schematische Detailansicht des Chirurgieroboters aus Fig. 6A;
- Fig. 6C: eine weitere schematische Detailansicht des Chirurgieroboters aus Fig. 6A;
- Fig. 7: eine schematische Darstellung eines Gehäuses eines Chirurgieroboters;
- Fig. 8: eine schematische Darstellung eines Fixierelements für einen Chirurgieroboter;
- Fig. 9A bis 9G: schematische Darstellungen des Ablaufs der Montage eines im Körper eines Patienten montierbaren Chirurgieroboters;
- Fig. 10A: eine schematische Ansicht eines abwinkelbaren Roboterarms mit einer großen Abwinklung eines distalen Armsegments und einer großen Biegung eines flexiblen Armsegments des distalen Armsegments; und
- Fig. 10B: eine schematische Ansicht des abwinkelbaren Roboterarms aus Fig. 10A mit einer geringen Abwinklung des distalen Armsegments und einer geringen Biegung des flexiblen Armsegments des distalen Armsegments.

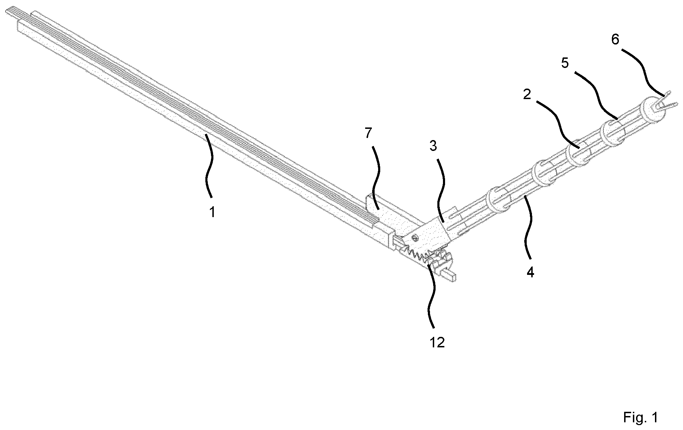

Die Fig. 1 zeigt eine Ausführung eines abwinkelbaren Roboterarms für die minimal-invasive Chirurgie. Der Roboterarm ist mit einem proximalen Armsegment 1 und einem distalen Armsegment 2 gebildet. Hierbei ist das proximale Armsegment 1 als steifes Segment gebildet. Das distale Armsegment 2 ist mit einem festen Segment 3 und einem hieran distal anschließenden flexiblen Armsegment 4 gebildet. Das flexible Armsegment 4 weist fünf NiTi-Drähte als Zug-Schub-Mittel 5 auf. Hierbei sind um eine zentrale Achse des flexiblen Armsegments 4 zwei jeweils antagonistisch wirkende Paare von Zug-Schub-Mitteln 5 angeordnet. Ein Zug an einem der Zug-Schub-Mittel 5 eines Paares bei gleichzeitigem Schub an dem anderen Zug-SchubMittel 5 des Paares bewirkt eine Abwinklung in Form einer Biegung des flexiblen Armsegments 4. Mittels Schub und Zug an einem auf der zentralen Achse des flexiblen Armsegments 4 verlaufenden Zug-Schub-Mittels 5 wird eine Endeffektor 6 des Roboterarms betätigt, bei dem es sich in dem Ausführungsbeispiel der Fig. 1 um ein Zangenelement handelt, dessen Zangenbacken geöffnet und geschlossen werden, um eine Greifbewegung bereitzustellen.

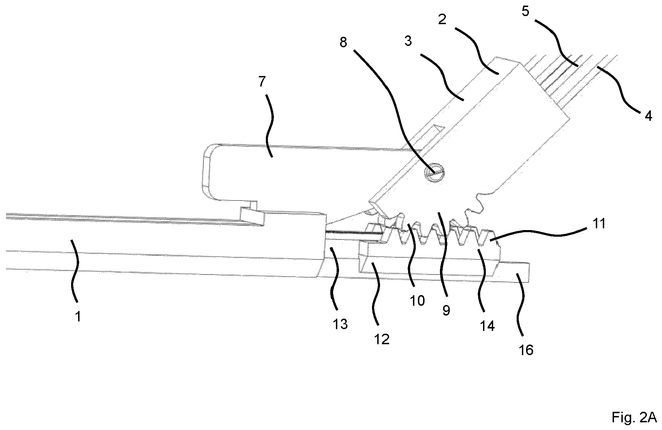

Wie in der Detailansicht der Fig. 2A zu erkennen, sind das proximale Armsegment 1 und das distale Armsegment 2 sind abwinkelbar miteinander verbunden. Hierzu sind zwei Flanken des festen Segments 3 um eine Verbindungs-Ausformung 7 herum angeordnet und mittels einer Schraubverbindung 8 durch die Flanken und das die Verbindungs-Ausformung 7 hindurch mit der Verbindungs-Ausformung schwenkbar verbunden. Durch die Schraubverbindung 8 ist somit eine Schwenkachse des distalen Armsegments 2 gegenüber dem proximalen Armsegment 1 definiert. Für ein Abwinkeln des distalen Armsegments 2 gegenüber dem gegenüber dem proximalen Armsegment 1 weist das distale Armsegment 2 zwei aus den Flanken geformte Ritzelsegmente 9 auf, welche mit Ritzelzähnen 10 gebildet sind, wobei die Ritzelzähne 10 einem Ritzel entsprechend um die Schwenkachse angeordnet sind. Stangenzähne 11 eine Zahnstangenelements 12 greifen in die Ritzelzähne. Hierdurch kann mittels einer Linearbewegung des Zahnstangenelements 12 eine Abwinklung des distalen Armsegments 2, nämlich des festen Segments 3, relativ zu dem proximalen Armsegment 1 um die Schwenkachse erreicht werden, wobei die Linear- oder translatorische Bewegung der Stangezähne 11 über den Eingriff mit den Ritzelzähnen 10 eine Rotation der Ritzelsegmente 9 und damit des festen Segments 3 um die Schwenkachse bewirken. Diese Abwinklung des distalen Armsegments 2 ist von einer Abwinklung durch eine Biegung des flexiblen Armsegments 4 prinzipiell unabhängig, wobei jedoch durch eine relative Wegänderung der Zug-Schubmittel 5 beim Abwinkeln des festen Segments 3 ein Einfluss auf das flexible Armsegment 4 entstehen kann.

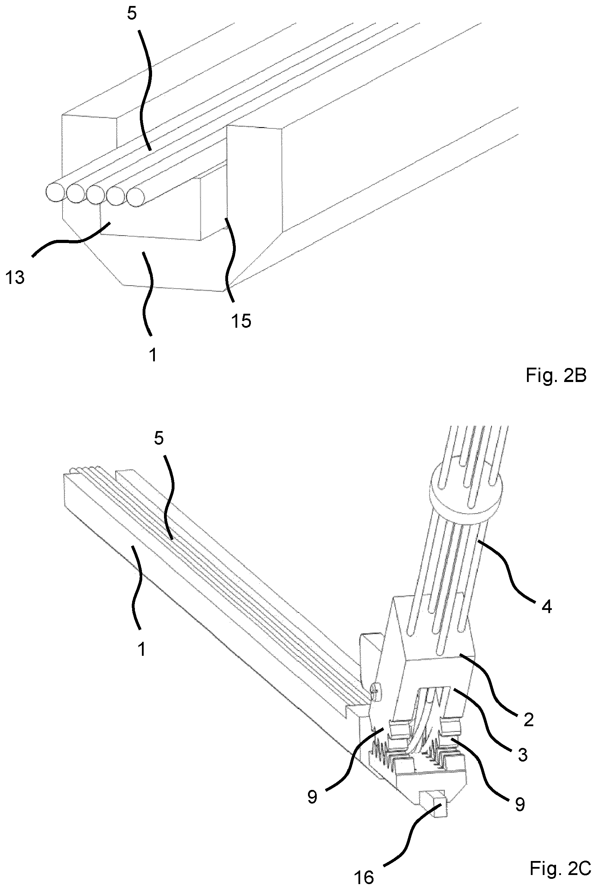

Die Detailansichten der Fig. 2B und 2C veranschaulichen die Abwinklung des distalen Armsegments 2 gegenüber dem proximalen Armsegment 1 und die lineare Führung des Zahnstangenelements 12 während der Abwinklung. Hierbei zeigt die Fig. 2B eine Sicht auf das proximale Ende des proximalen Armsegments 1. Die Fig. 2C stellt eine Sicht auf das distale Ende des proximalen Armsegments 1 mit dem hieran schwenkbar angeordneten distalen Armsegment 2 dar. Hierbei ist zu erkennen, dass die Zug-Schub-Elemente 5 des flexiblen Armsegments 4 über die gesamte Länge des proximalen Armsegments 1 bis zu dessen proximalen Ende geführt werden. Hier können Linearaktoren mit den Zug-Schub-Elementen 5 wirkverbunden sein, um diese linear zu bewegen und so das flexible Armsegment 4 zu aktuieren.

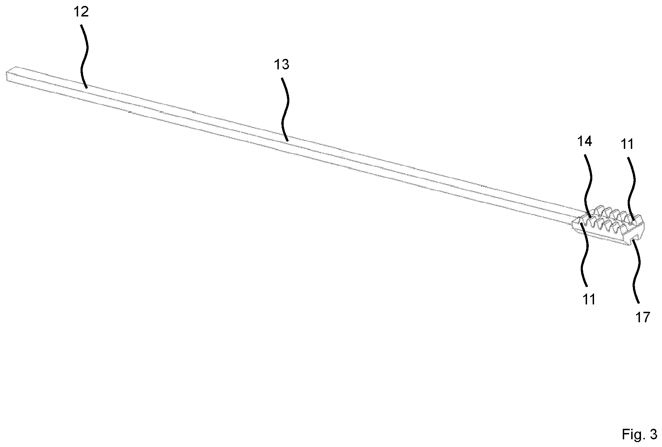

Wie in der Fig. 3 gezeigt, ist das Zahnstangenelement 12 mit einem schmalen proximalen Zahnstangensegment 13 und einem breiteren distalen Zahnstangensegment 14 gebildet. Hierbei sind an dem distalen Zahnstangensegment 14 an gegenüberliegenden Seiten zwei Sätze von Stangenzähnen 11 gebildet, welche in dem abwinkelbaren Roboterarm jeweils in Eingriff mit den Ritzelzähnen 10 eines der Ritzelsegmente 9 des distalen Armsegments 2 stehen.

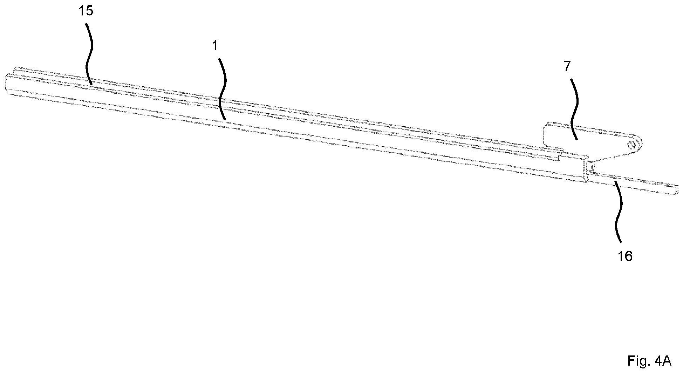

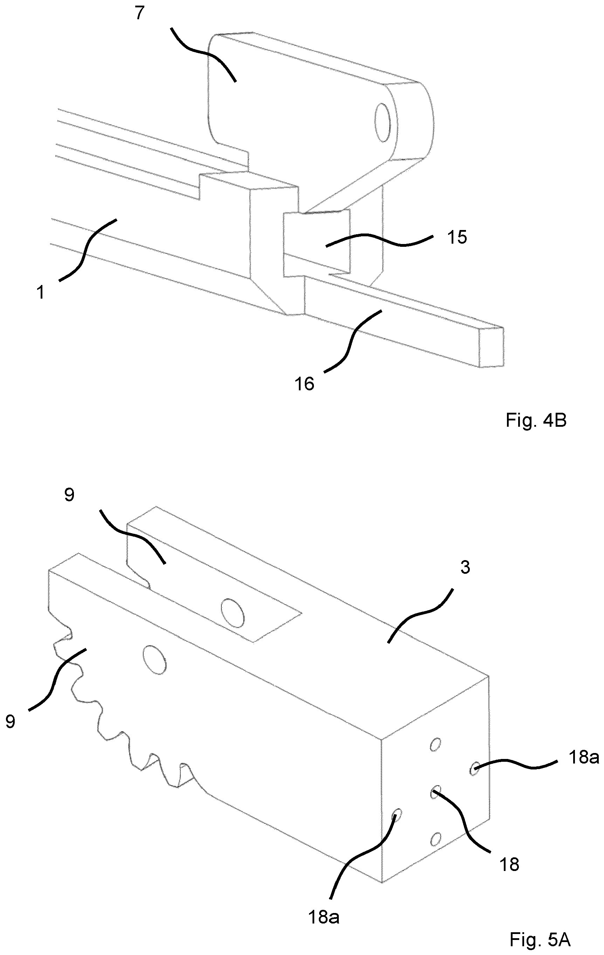

Die Fig. 4A zeigt das proximale Armsegment 1. Die Fig. 4B ist eine Detailansicht des distalen Endes des proximalen Armsegments 1. In dem proximalen Armsegment 1 ist ein Führungskanal 15 gebildet, welche zur linearen Führung des Zahnstangenelements 12 in Längsrichtung des proximalen Armsegments 1 das schmale proximale Zahnstangensegment 13 aufnimmt. Der Führungskanal 15 ist mit einem einer U-Form angenäherten Querschnitt gebildet und der Querschnitt des proximalen Zahnstangensegments 13 ist an den Querschnitt des Führungskanals 15 angepasst, wie beispielsweise in der Fig. 2B zu erkennen ist.

Zusätzliche weist das proximale Armsegment 1 einen Führungsstab 16 auf, welcher in einem Zahnstangenelement-Führungskanal 17 lineargeführt ist, wodurch eine zusätzliche Führung der translatorischen Bewegung des Zahnstangenelements 12 zur Abwinklung des distalen Armsegments 2 bereitgestellt ist. Der Führungsstab 16 ist an dem distalen Ende des proximalen Armsegments 1 angeordnet und steht sich in Längsrichtung erstreckend von diesem hervor. Der Zahnstangenelement-Führungskanal 17 ist an der Unterseite des breiten distalen Zahnstangensegment 14 gebildet und die Querschnittsformen des Führungsstabs 16 und des Zahnstangenelement-Führungskanals 17 sind derart aufeinander abgestimmt, dass das Zahnstangenelement 12 mit dem Zahnstangenelement-Führungskanal 17 auf dem Führungsstab 16 im Sinne einer Linearführung laufen kann, wie beispielsweise in Fig. 2A und 2C zu erkennen. In dem gezeigten Ausführungsbeispiel weisen das Zahnstangenelement 12 und der Zahnstangenelement-Führungskanal 17 rechteckige Querschnitte auf. Alternativ sind auch andere Querschnittsformen geeignet, beispielsweise eine U- oder V-Form.

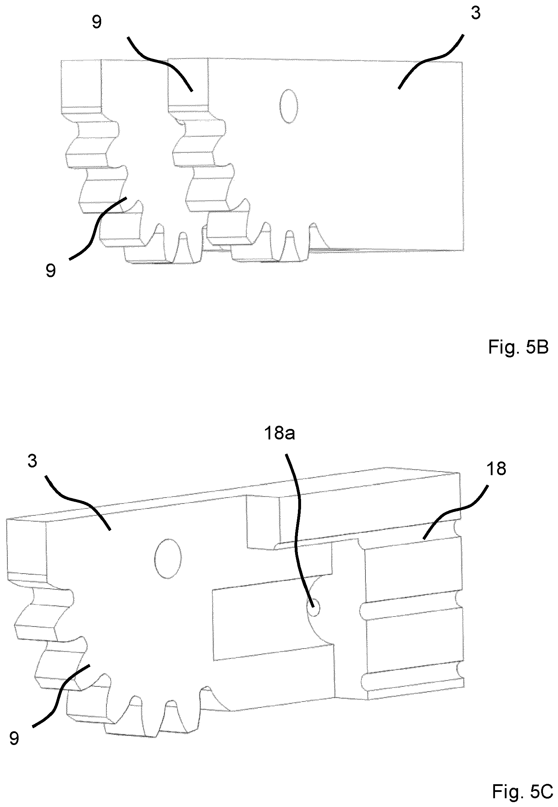

Die Fig. 5A und 5B zeigen ein festes Segment 3 eines distalen Armsegments 2 aus unterschiedlichen Perspektiven. An einer in Längsrichtung den an den Flanken gebildeten Ritzelsegmenten 9 gegenüberliegenden Seite des festen Segments sind Führungsbohrungen 18 gebildet, welche die Zug-Schub-Elemente 5 des flexiblen Armsegments 4 aufnehmen und linear führen. Durch die Führungsbohrungen werden die Zug-Schub-Elemente 5 von dem flexiblen Armsegment 4 zu dem proximalen Armsegment 1 und wie in den Fig. 2A, 2B und 2C erkennbar durch dieses zu dessen proximalen Ende geführt. An dem proximalen Ende des proximalen Armsegments 1 können die Zug-Schub-Elemente 5 dann mit Linearaktoren verbunden sein, um das flexible Armsegment 4 anzutreiben und zu steuern.

Die Fig. 5C zeigt eine Schnittansicht des festen Segments 3, in der zu erkennen ist, dass es sich bei den Führungsbohrungen 18 um Durchgangsbohrungen handelt. Hierbei verlaufen zwei Führungsbohrungen 18a der gezeigten Ausführungsform teilweise durch die Flanken mit den Ritzelsegmenten 9, beziehungsweise münden in eine Aussparung in den Flanken. In der Fig. 5C ist erkennbar, dass die betreffenden Zug-Schub-Elemente 5 in diesen Führungsbohrungen 18a durch eine distale Wand des festen Segments 3 geführt und anschließend durch eine leichte Biegung von der jeweiligen Flanke weg geführt werden können.

Bei einer Abwinklung des festen Segments 3 gegenüber dem proximalen Armsegment 1 kann sich für einige der Zug-Schub-Elemente 5, je nach deren Führung entlang des Roboterarms, ein Weg vom proximalen Ende des proximalen Armsegments 1 bis zum distalen Ende des festen Segments 3 geringfügig ändern, was wiederum zu einer geringfügigen Aktuierung des flexiblen Armsegments 4 führen kann. Eine solche geringfügige Aktuierung des flexiblen Armsegments 4 kann entweder in einer Gesamtbewegung des Roboterarms berücksichtigt und genutzt werden oder es kann vorgesehen sein, durch eine entsprechende aktuierte Gegenbewegung des betreffenden Zug-Schub-Elements die Wegänderung auszugleichen und so eine ungewollte Aktuierung des flexiblen Armsegments 4 zu vermeiden.

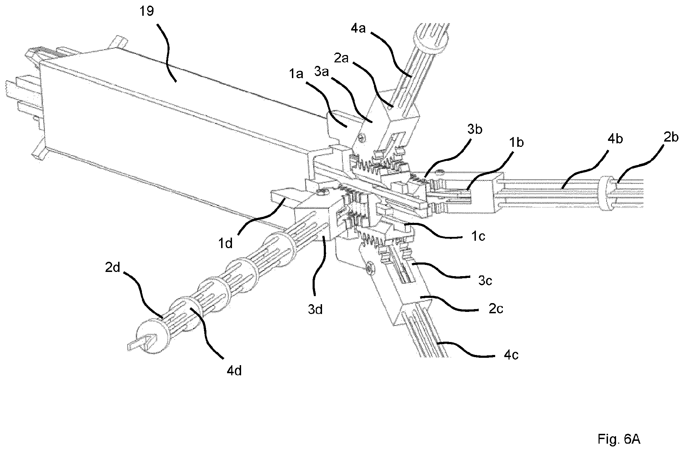

Die Fig. 6A zeigt einen Chirurgieroboter mit vier offenbarungsgemäßen abwinkelbaren Roboterarmen. Hierbei sind die die vier proximalen Armsegmente 1a, 1b, 1c, 1d der Roboterarme über den überwiegenden Teil ihrer jeweiligen Längserstreckung in einem umhüllenden Gehäuse 19 angeordnet. Die distalen Armsegmente 2a, 2b, 2c, 2d mit den festen Segmenten 3a, 3b, 3c, 3d und den flexiblen Armsegmenten 4a, 4b, 4c, 4d sind außerhalb des Gehäuses 19 angeordnet und können sich während einer Operation mit dem Chirurgieroboter bewegen.

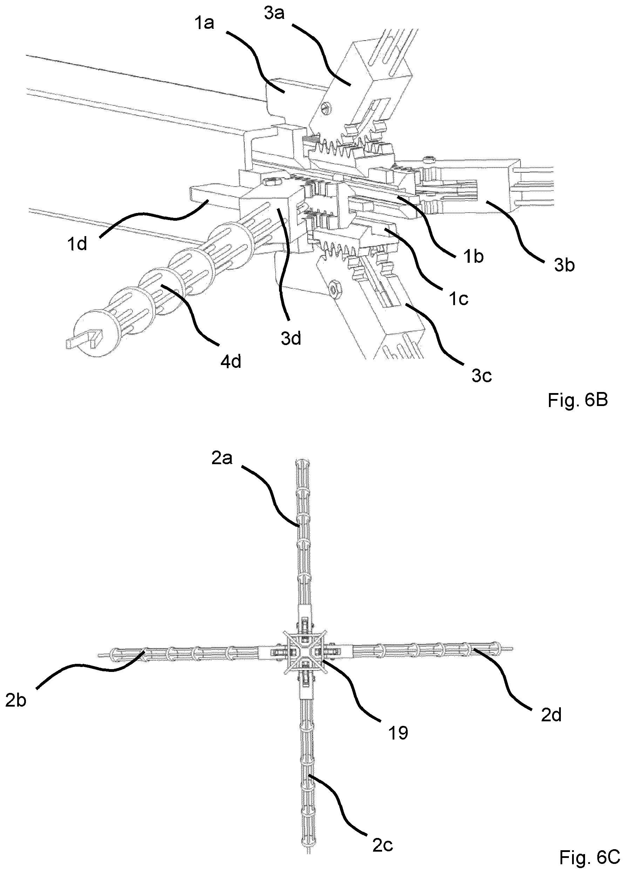

In der Fig. 6B ist eine Abwinklung der jeweiligen festen Segmente 3a, 3b, 3c, 3d gegenüber den jeweiligen proximalen Armsegmenten 1a, 1b, 1c, 1d im Detail gezeigt. Durch eine Abwinklung der jeweiligen festen Segmente 3a, 3b, 3c, 3d können die flexiblen Armsegmente 2a, 2b, 2c, 2d zunächst auseinanderbewegt werden und anschließend durch eine Biegung der flexiblen Armsegmente 2a, 2b, 2c, 2d deren Endeffektoren wieder am Operationssitus aus unterschiedlichen Richtungen zueinander gebracht werden, wodurch Operationsbewegung im Rahmen einer sogenannten Triangulation gegenüber einer parallelen Führung der Roboterarme an den Operationssitus erleichtert sein können.

Die Fig. 6C zeigt den Chirurgieroboter aus einer Perspektive von einem proximalen Ende aus. Das proximale Ende des Chirurgieroboters kann zur Aktuierung der Roboterarme an weitere Komponenten angeschlossen werden, insbesondere an eine Antriebseinheit mit Aktoren für den Antrieb der verschiedenen Freiheitsgrade der Roboterarme.

Der Chirurgieroboter ist in der gezeigten Ausführungsform ein im Körper eines Patienten montierbarer Roboter. Hierbei werden die abwinkelbaren Roboterarme einzeln in den Körper des Patienten eingebracht und erst im Anschluss miteinander in dem Gehäuse 19 zu dem Chirurgieroboter verbunden.

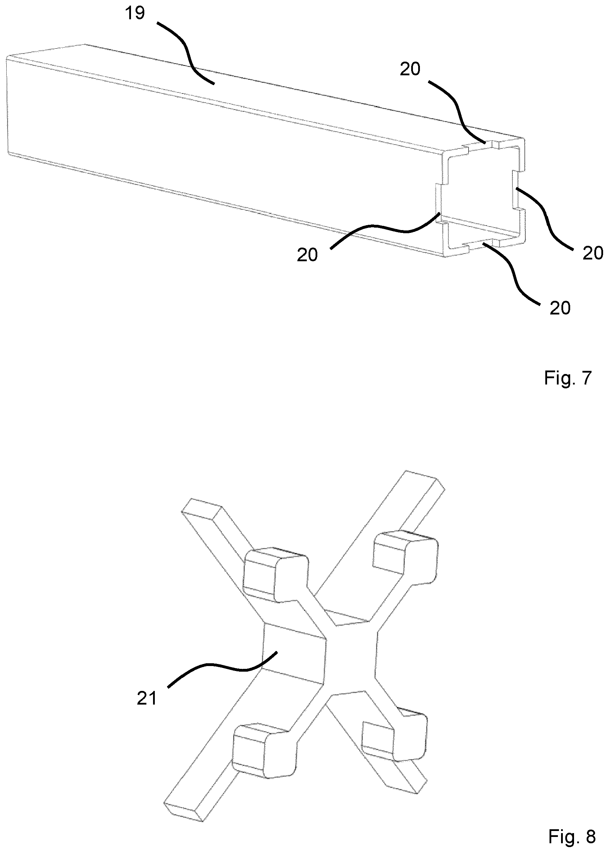

Die Fig. 7 ist eine Detailansicht des Gehäuses 19 des Chirurgieroboters. Hierbei ist erkennbar, dass das Gehäuse 19 an einem distalen Ende Aussparungen 20 aufweist, welche der Montage des Chirurgieroboters dienen, wie nachfolgend im Einzelnen erläutert.

Die Fig. 8 zeigt ein dichtendes Fixierelement 21, welches nach dem Anordnen mehrerer Roboterarme in dem Gehäuse 19 in ein proximales Ende des Gehäuses eingefügt wird, um die Roboterarme in dem Gehäuse korrekt zueinander zu positionieren und in dieser Position zu fixieren. Gleichzeitig wirkt das Fixierelement 21 dichtend. Hierdurch kann insbesondere vermieden sein, dass im Einsatz, also während einer Operation, Insufflationsgas aus der Bauchhöhle des Patienten zwischen den Roboterarmen durch das Gehäuse 19 hindurch austritt. Beispielsweise kann das Fixierelement 21 zur Dichtung mit einer Gummierung versehen sein.

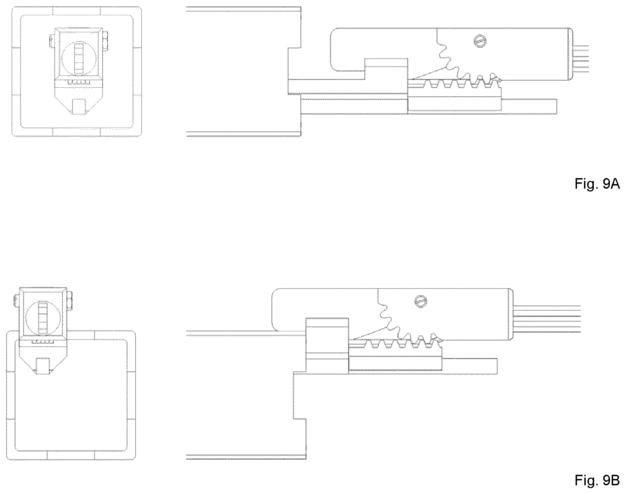

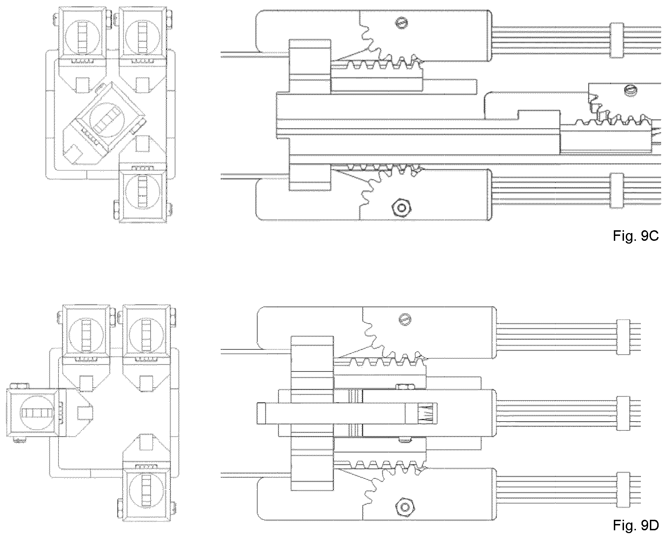

In den Figuren 9A bis 9G ist ein Montageprozess des Chirurgieroboters dargestellt. Hierbei werden die Roboterarme nacheinander in das distale Ende des Gehäuses 19 eingeführt. Wie in den Figuren 9A und 9B erkennbar, wird der erste Roboterarm mittig in das Gehäuse 19 eingeführt und anschließend an den Rand des Gehäuses 19 bewegt. So können die weiteren Roboterarme ebenfalls in das Gehäuse 19 eingeführt und an den Rand des Gehäuses 18 bewegt werden. Das Einführen des vierten Roboterarms erfordert eine Verdrehung, um diesen durch den Hohlraum zwischen den drei bereits eingeführten Roboterarmen einzuführen und anschließen an den Rand des Gehäuses 19 zu bewegen Dies ist in den Figuren 9C und 9D im Einzelnen erkennbar.

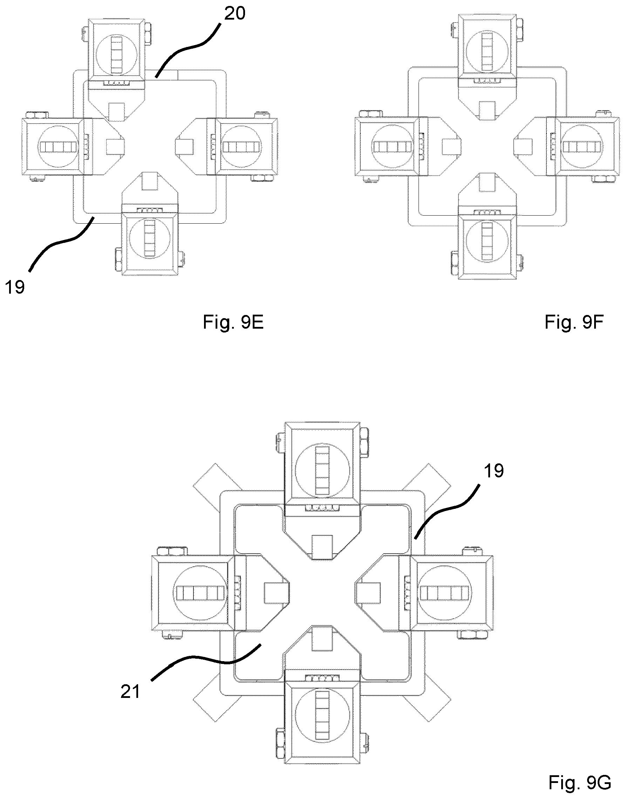

Im Anschluss findet eine Umordnung der Roboterarme in dem Gehäuse 19 statt, wie in den Figuren 9E und 9F gezeigt, sodass jeder der Roboterarme in einer zugeordneten montierten Stellung angeordnet ist. Beim Anordnen der Roboterarme am Rand des Gehäuses 19 wird, wie in den Figuren 9A bis 9D erkennbar, die jeweilige Verbindungs-Ausformung 7 des betreffenden Roboterarms über den Rand Gehäuses 19 geschoben, wodurch der Roboterarm mit dem Rand des Gehäuses verbunden ist. Nach dem Umordnen der Roboterarme werden die jeweiligen Verbindungs-Ausformungen 7 in die zugeordneten Aussparungen 20 geschoben, wodurch die Roboterarme am distalen Ende des Gehäuses 19 in ihrer montierten Position fixiert werden.

In alternativen Ausgestaltungen können die Roboterarme derart miniaturisiert sein, dass sie direkt in ihrer finalen Anordnung in das Gehäuse 19 eingeführt werden können. In diesem Fall kann der Schritt des Umordnens bei der Montage entfallen.

Abschließend wird eine das dichtende Fixierelement 21 in dem proximalen Ende des Gehäuses 19 angeordnet, wie in der Fig. 9G gezeigt, wobei die Fig. 9G eine Sicht auf das distale Ende des Gehäuses 19 darstellt, so dass das Fixierelement 21 teilweise verdeckt dargestellt ist. Durch das Fixierelement werden sowohl die Roboterarme in ihrer montierten Anordnung am proximalen Ende des Gehäuses 19 fixiert als auch mittels der Dichtung eine Insufflation während einer Operation aufrechterhalten.

In alternativen Ausgestaltungen kann ein Chirurgieroboter bereitgestellt sein, der nicht im Körper eines Patienten montierbar ist. Beispielsweise können die Roboterarme an Armen von als solches bekannten Robotern angeordnet werden, welche auch während einer Operation vollständig außerhalb des Patienten verbleiben und zusätzliche Freiheitsgrade bereitstellen.

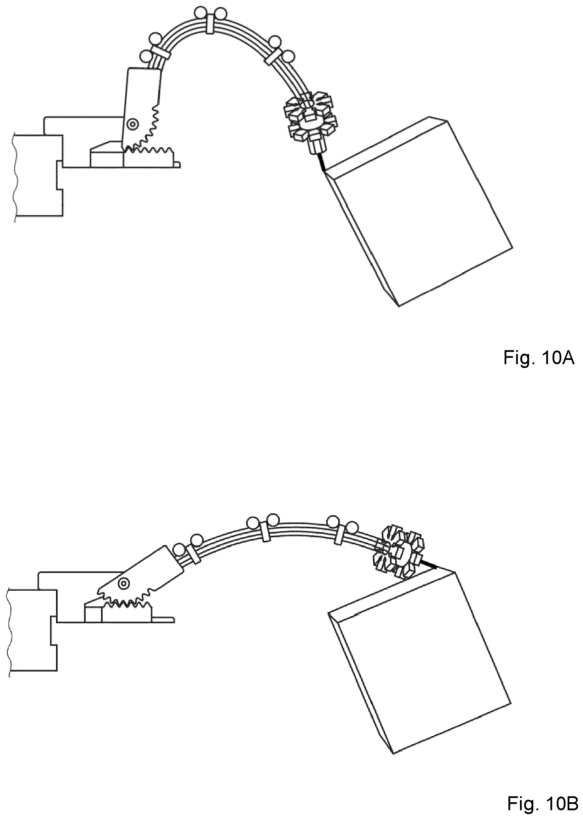

In den Fig. 10A und 10B ist eine Ausführungsforme eines abwinkelbaren Roboterarms für die minimal-invasive Chirurgie in unterschiedlichen Bewegungszuständen gezeigt. Gemäß der Fig. 10A ist das feste Segment 3 gegenüber dem proximalen Armsegment 1 in einem annähernd rechten Winkel abgewinkelt. Hierbei ist zu erkennen, dass das Zahnstangenelement 12 weit vorgeschoben ist. Zusätzlich ist das flexible Armsegment 4 stark gebogen. Das distale Ende des Roboterarms hält ein Gewicht. Im Vergleich zu dem in der Fig. 10A gezeigten Zustand ist gemäß der Fig. 10B das feste Segment 3 gegenüber dem proximalen Segment um einen deutlich geringeren Winkel abgewinkelt und das flexible Armsegment 4 ist weniger stark gebogen.

Mit einer beispielhaften Ausführung eines abwinkelbaren Roboterarms wurden Versuche zur Belastbarkeit durchgeführt. Dabei entsprachen der Aufbau des Roboterarms und die Bewegungsformen den Darstellungen der Fig. 10A und 10B, wobei das proximale Armsegment 1, das feste Segment 3 sowie das Zahnstangenelement 12 mittels 3D-Druck hergestellt wurden. Der Roboterarm besteht aus proximalem Armsegment, distalem Armsegment mit festem Segment, einer Schraubenverbindung, einer Zahnstange und einem flexiblen Armsegment. Ein Ritzel ist in das distale Armsegment integriert. Die Zähne der Zahnstange und die Zähne des Ritzels greifen ineinander. Dabei ist die Zahnstange mittels eines Führungskanals geführt. Das flexible Armsegment besteht aus Scheiben. Die Scheiben haben zwei Funktionen: Sie stellen den radialen Abstand zwischen einzelnen NiTi-Drähten sicher und durch die Befestigung aller Drähte am Ende des flexiblen Armsegments kann eine Biegung erzielt werden. Eine Endscheibe ist beidseitig an jedem NiTi-Draht befestigt.

In den Versuchen wies die Winkelverstelleinheit eine hohe Funktionalität auf. Als schwächster Punkt erwiesen sich die NiTi-Drähte. Die Verzahnung zwischen Zahnstange und Ritzel war zu jedem Zeitpunkt einwandfrei. Obwohl der Winkelverstellmechanismus einer doppelten Belastung durch die Biegung der NiTi-Drähte als auch das Heben einer Last mit einer Masse von 30 g ausgesetzt war und die Zähne mit einer Höhe von ca. 1,5 mm relativ flach sind, war die Kraftübertragung zu jedem Zeitpunkt gegeben. Das minimale Spiel zwischen Ritzelzähnen und Zahnstangenzähnen war ausgereichend, um einen reibungslosen Bewegungsablauf zu gewährleisten. Durch die Führung per Führungsschiene war eine positionsunabhängige Stabilität der Zahnstange erreicht.

Bei einer Anbringung des Gewichts direkt an dem Ende des distalen Armsegments, also an einer Grundplatte, konnten problemlos Lasten von 200 g gehoben werden. Hieraus kann geschlossen werden, dass der Abwinklungsmechanismus für die Verwendung in der minimal-invasiven Chirurgie geeignet ist. Hierbei war es unerwartet, dass die Kombination aus Zahnstange, Ritzel und Führungsschiene selbst bei einer hohen Last von 200 g als Winkelverstellmöglichkeit die festgestellte Funktionalität aufweist. Die Verzahnung erwies sich als zuverlässig und Verstellbefehle wurden umgesetzt.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Abwinkelbarer Roboterarm für die minimal-invasive Chirurgie, mit
- einem proximalen Armsegment (1), welches sich entlang einer Längsachse des Roboterarms erstreckt und
- einem distalen Armsegment (2), welches mit dem proximalen Armsegment (1) um eine zu der Längsachse orthogonale Schwenkachse schwenkbar verbunden ist,
wobei
- das distale Armsegment (2) ein Ritzelsegment (9) aufweist, welches mit in Form eines Ritzels um die Schwenkachse angeordneten Ritzelzähnen (10) gebildet ist; und
- ein Zahnstangenelement (12) an dem proximalen Armsegment (1) derart angeordnet ist, dass
- das Zahnstangenelement (12) in Längsrichtung des Zahnstangenelements (12) parallel zu der Längsachse mittels einer Translation beweglich ist, und
- an dem Zahnstangenelement (12) gebildete Stangenzähne (11) mit den Ritzelzähnen (10) in Eingriff stehen, so dass eine Bewegung des Zahnstangenelements (12) in der Längsrichtung über die Stangenzähne (11) und die Ritzelzähne (10) in eine Rotationsbewegung des distalen Armsegments (2) um die Schwenkachse gewandelt wird und das distale Armsegment (2) relativ zu dem proximalen Armsegment (1) um die Schwenkachse verschwenkt.

2. Abwinkelbarer Roboterarm nach Anspruch 1, wobei die Ritzelzähne (10) als Ausformungen des distalen Armsegments (2) gebildet sind.

3. Abwinkelbarer Roboterarm nach Anspruch 1 oder 2, wobei das distale Armsegment (2) ein zweites Ritzelsegment (9) mit um die Schwenkachse angeordneten Ritzelzähnen (10) aufweist, welches von dem Ritzelsegment (9) entlang der Schwenkachse beabstandet an dem distalen Armsegment (2) angeordnet ist.

4. Abwinkelbarer Roboterarm nach Anspruch 3, wobei das Zahnstangenelement (12) zwei Sätze von Stangenzähnen (11) aufweist, wobei die zwei Sätze von Stangenzähnen (11) parallel und in Richtung der Schwenkachse beabstandet zueinander angeordnet sind, derart, dass der erste Satz von Stangenzähnen (11) mit den Ritzelzähnen (10) des Ritzelsegments (9) in Eingriffs steht und der zweite Satz von Stangenzähnen (11) mit den Ritzelzähnen (10) des zweiten Ritzelsegments (9) in Eingriff steht.

5. Abwinkelbarer Roboterarm nach mindestens einem der vorangehenden Ansprüche, wobei das Zahnstangenelement (12) mit einem proximalen Zahnstangensegment (13) und einem in der Längsrichtung an das proximale Zahnstangensegment (13) anschließendem distalen Zahnstangensegment (14) gebildet ist, wobei das distale Zahnstangesegment in Richtung der Schwenkachse breiter ist als das proximale Zahnstangensegment (13).

6. Abwinkelbarer Roboterarm nach mindestens einem der vorangehenden Ansprüche, wobei das proximale Armsegment (1) einen entlang der Längsachse verlaufenden Führungskanal (15) aufweist, in welchem das Zahnstangenelement (12), insbesondere mit einem proximalen Zahnstangensegment (13), zumindest abschnittsweise angeordnet und geführt ist.

7. Abwinkelbarer Roboterarm nach mindestens einem der vorangehenden Ansprüche, wobei
- das proximale Armsegment ein Armsegment-Führungselement aufweist und
- das Zahnstangenelement einen Zahnstangenelement-Führungskanal aufweist, wobei das Armsegment-Führungselement zumindest abschnittsweise in dem Zahnstangenelement-Führungskanal angeordnet und geführt ist.

8. Abwinkelbarer Roboterarm nach mindestens einem der vorangehenden Ansprüche, wobei das distale Armsegment (2) zumindest abschnittsweise als flexibles Armsegment (4) gebildet ist, welches entlang seiner Längserstreckung kontinuierlich oder quasi-kontinuierlich abwinkelbar ist.

9. Abwinkelbarer Roboterarm nach mindestens Anspruch 8, wobei das flexible Armsegment (4) mit mindestens drei Zug-Schub-Mitteln (5) gebildet ist, welche entlang einer Längserstreckung des flexiblen Armsegments (4) parallel und entlang der Längserstreckung verschiebbar zueinander angeordnet sind.

10. Abwinkelbarer Roboterarm nach mindestens einem der vorangehenden Ansprüche, wobei das distale Armsegment (2) zumindest abschnittsweise als diskret-robotisches Armsegment mit entlang seiner Längserstreckung hintereinander angeordneten Segmenten gebildet ist, wobei das diskret-robotische Armsegment entlang seiner Längserstreckung segmentweise abwinkelbar ist.

11. Abwinkelbarer Roboterarm nach mindestens einem der vorangehenden Ansprüche, mit einem Linearaktor, welcher mit dem Zahnstangenelement (12) verbunden und eingerichtet ist, das Zahnstangenelement (12) parallel zu der Längsachse translatorisch vorund zurückzubewegen.

12. Chirurgieroboter, insbesondere für die minimal-invasive Chirurgie, mit einem abwinkelbaren Roboterarm nach einem der vorangehenden Ansprüche.

13. Chirurgieroboter nach Anspruch 12, wobei der Chirurgieroboter als im Körper eines Patienten montierbarer Roboter ausgebildet ist.

14. Verfahren zum Herstellen eines abwinkelbaren Roboterarms, mit den Schritten:
- Bereitstellen eines proximalen Armsegments (1), dessen Erstreckungsrichtung eine Längsachse des Roboterarms definiert;
- Bereitstellen eines distalen Armsegments (2) mit einem Ritzelsegment (9), welches mit in Form eines Ritzels um eine Schwenkachse angeordneten Ritzelzähnen (10) gebildet ist;
- Verbinden des distalen Armsegments (2) mit dem proximalen Armsegment (1), derart, dass das distale Armsegment (2) um die Schwenkachse relativ zu dem proximalen Armsegment (1) schwenkbar ist und die Schwenkachse zu der Längsachse orthogonal verläuft; und
- Anordnen eines Zahnstangenelements (12) an dem proximalen Armsegment (1), derart, dass
- das Zahnstangenelement (12) in Längsrichtung des Zahnstangenelements (12) parallel zu der Längsachse mittels einer Translation beweglich ist, und
- an dem Zahnstangenelement (12) gebildete Stangenzähne (11) mit den Ritzelzähnen (10) in Eingriff stehen, so dass eine Bewegung des Zahnstangenelements (12) in der Längsrichtung über die Stangenzähne (11) und die Ritzelzähne (10) in eine Rotationsbewegung des distalen Armsegments (2) um die Schwenkachse gewandelt wird und das distale Armsegment (2) relativ zu dem proximalen Armsegment (1) um die Schwenkachse verschwenkt.
